# EUROPEAN PATENT SPECIFICATION

(11) **EP 2 999 445 B1**
(45) Date of publication and mention of the grant of the patent: **01.04.2020**
(21) Application number: 13725044.5
(22) Date of filing: 20.05.2013
(51) Int. Cl.: A61F 13/04

(54) **HIP SPICA CAST AND UNDERGARMENT FOR USE WITH HIP SPICA CAST**
HÜFTSPICA-GIPS UND UNTERWÄSCHE ZUR VERWENDUNG MIT DEM HÜFTSPICA-GIPS
SPICA PLÂTRÉ POUR HANCHE ET SOUS-VÊTEMENT POUR UTILISATION AVEC UN SPICA PLÂTRÉ POUR HANCHE

(43) Date of publication of application: 30.03.2016
(73) Proprietor: BSN Medical, Inc., Charlotte, NC 28209-4633 (US)
(72) Inventor: EVANS, John, C., Lancashire Nr Rochdale OL 16 3RF (GB); JORISSEN, Koen, Jozef Maria, Hamburg (DE)
(74) Representative: Algemeen Octrooi- en Merkenbureau B.V.
(86) International application number: PCT/US2013/041802
(87) International publication number: WO 2014/189483

(56) References cited:
- WO-A1-93/10732
- WO-A2-2010/131840
- US-A1- 2005 043 664
- US-A1- 2012 220 908
- "Application Manual Hip Spica Cast", , 2011, pages 1-20, XP055097166, Retrieved from the Internet: URL:http://www.bsnmedical.com/fileadmin/z- countries/0-Global/PDF/delta_dry_hip.pdf [retrieved on 2014-01-20] cited in the application

## Description

### Technical Field and Background of the Invention

This invention relates to an undergarment formed of orthopedic padding, for example, for use as an undercast padding of the type used to protect and cushion the skin of a patient from the relatively rigid material of a cast, such as those constructed of plaster of Paris or synthetic cast tape. Specifically, this invention relates to a hip spica cast intended primarily for infants and other pediatric patients. The cast is constructed of an undercast padding material characterized by having a very open water and air transmissive structure enclosed within a very open water and air transmissive cast tape.

A cast which includes the torso of the body and one or more limbs is called a spica cast. A hip spica includes the torso of the body and one or more legs. A hip spica which covers only one leg to the ankle or foot may be referred to as a single hip spica, while one which covers both legs is called a double hip spica. A one-and-a-half hip spica encases one leg to the ankle or foot and the other to just above the knee. The extent to which the hip spica covers the torso depends greatly on the injury and the surgeon; the spica may extend only to the navel, allowing mobility of the spine and the possibility of walking with the aid of crutches, or may extend to the rib cage or even to the armpits in some rare cases. Hip spicas are used for a number of conditions and injuries, such as congenital hip dislocation or dysplasia.

In some cases, a hip spica may only extend down one or more legs to above the knee. Such casts, called pantaloon casts, are used to immobilize an injured lumbar spine or pelvis, in which case the torso portion of the cast usually extends to or just below the armpits. The specific example discussed in this application is such a pantaloon cast, but the invention is not limited to this particular type of cast.

A traditional hip spica cast is constructed from a simple stockinette and padding material made from cotton or synthetic fibers, and offers poor or no water resistant capability. Cotton and some synthetic padding actually absorb and retain large quantities of water. A hip spica cast is typically worn for a period of 6-10 weeks. During this period of time, traditional casts having a water-absorbent stockinette can promote skin maceration and discomfort. This is a particular problem with infants and very small children who are incontinent and therefore are far more likely to soil the cast with urine and feces. To facilitate toileting or diaper changing and hygienic cleaning, an opening, referred to as the "perineal opening", is typically created in the cast at the groin. It is formed either during cast application or after cast application by cutting the hole with a cast saw. The opening must then be petalled or lined to keep this area of the cast clean and dry. However, this is difficult, particularly with infants and small children. In reality, within a relatively short period of time after traditional hip spica cast application, the area around the perineal opening becomes soiled with urine and feces and develops foul odors that must be tolerated or masked with various deodorizers for a month or more. Rashes, maceration, skin and systemic infections, open sores and other conditions may retard the treatment schedule and impose pain and discomfort on the infant or pediatric patient. US2005/043664 A1, which is regarded as the closest prior art, discloses a prefabricated sleeve of a fabric material which can be formed as a single unit prior to positioning on a body part or limb. BSN medical Delta-Dry® application manual hip spica cast discloses a hip spica cast comprising Delta-Dry® padding material . This material can be wrapped around the torso and the legs of the patient before cast tape is applied.

The present invention provides a more conformable, water-resistant product that permits the material to be formed into an anatomically-shaped and sized undergarment that can be donned onto the patient, and then enclosed by wrapping with a suitable water and air permeable synthetic cast tape to form the spica cast.

One of the problems with conventional cast padding as well as commercially available water resistant padding is that the padding collapses underneath a cast over the duration of 4-6 weeks as water and perspiration are absorbed into the structure. This reduction in thickness and resultant increase in density retards moisture transfer by both wicking and evaporation, and lessens the protection offered by the padding.

The hip spica cast according to the present invention accommodates bathing, showering and contact with water for cleansing while permitting surface moisture to be dried, and moisture on the interior of the hip spica cast to evaporate relatively quickly, leaving the patient dry and comfortable. For smaller patients, the patient can be held in proximity to, for example, a handheld spray nozzle and the cast sprayed with a gentle spray of warm, cleansing water. Urine is readily washed away, and feces are dissolved, diluted and allowed to drain away. The patient is left clean and odor-free. Excess surface water is easily blotted away with an absorbent cloth or paper towel, and water on the interior evaporates within about one hour. This process can be repeated as necessary during the entire treatment period. Moreover, because of the ability to cleanse the patient as described above, it may not be necessary to provide the perineal opening in some instances.

### Summary of the Invention

Therefore, it is an object of the invention to provide a hip spica cast formed from a water and air permeable padding material formed into a garment, and enclosed within a water and air permeable synthetic cast tape. It is another object of the invention to provide an orthopedic padding that is comfortable when worn under a synthetic cast.

It is another object of the invention to provide a hip spica cast in the form of a garment that is relatively thin but still providing sufficient cushioning and thus provides a low profile undercast layer when properly overlapped during application.

It is another object of the invention to provide a hip spica cast that is relatively open and therefore breathable.

It is another object of the invention to provide a hip spica cast that is resistant to collapse during extended use.

It is another object of the invention to provide a hip spica cast that promotes drainage of water from the cast when wetting occurs.

It is another object of the invention to provide a breathable hip spica cast that is comfortable when worn against the skin under a wrapping of synthetic cast tape.

These and other objects of the present invention are achieved by providing an undergarment as set forth in independent claim 1. Preferred embodiments are defined in the dependent claims. Also provided is a hip spica cast comprising such an undergarment, as set forth in claim 7.

The undergarment has a torso portion including a top opening for being positioned on a torso portion of the patient and at first and second leg portions terminating in respective leg openings adapted to reside between the hips and knees of the patient, and wherein the undergarment is formed of first and second overlaid padding layers defining an anterior side of the undergarment and third and fourth overlaid padding layers defining a posterior side of the undergarment.

According to an embodiment of the invention, the first and second padding layers and the third and fourth padding layers are joined along left and right seam lines extending from a top opening to first and second leg openings at a lowermost terminus of the undergarment.

According to another embodiment of the invention, the monofilament yarns are thermoplastic and the seam lines are formed by ultrasonic energy or other suitable methods, such as by butt or overedge seaming, or with an adhesive, either in tape or atomized glue form.

According to another embodiment of the invention, the undergarment includes a perineal opening.

According to the invention, the undergarment is configured to form an undergarment for a pantaloon hip spica cast.

The cast tape is selected from the group consisting of a synthetic fiber or fiberglass/non-fiberglass substrate coated or saturated with a moisture-curable resin and stored in a flexible condition in a moisture impervious package until use, at which time removal of the cast tape from the moisture impervious package and exposure to moisture causes the substrate to harden over a time period sufficiently long enough to allow application of the cast tape while still flexible over the undergarment.

Also disclosed is an undergarment for use with cast tape to form a hip spica cast is provided, and includes at least one layer of a 3D spacer synthetic padding material formed into a knitted fabric of monofilament yarns and fabricated into an anatomically appropriate undergarment structure for being positioned around a torso and residing directly against the skin of a hip spica cast patient, the fabric for the padding material formed with at least 50 courses per meter preferably 200-850 courses per meter, and the padding material weighs between 50-400 g/m² and has a nominal thickness when not compressed or under tension of approximately 1.5-3.5 mm.

According to another embodiment of the invention, a hip spica cast is provided and includes an undergarment as described above, and a cast tape having a synthetic fiber or fiberglass/non fiberglass substrate coated or saturated with a moisture-curable resin and stored in a flexible condition in a moisture impervious package until use, at which time removal of the cast tape from the moisture impervious package and exposure to moisture causes the substrate to harden over a time period sufficiently long enough to allow application of the cast tape while still flexible over the undergarment. The cast tape is positioned circumferentially over the undergarment and around the torso of the patient.

### Brief Description of the Drawings

Some of the objects of the invention have been set forth above. Other objects and advantages of the invention will appear as the invention proceeds when taken in conjunction with the following drawings, in which:
Figure 1 is a perspective view of a hip spica cast undergarment according to one embodiment of the invention;
Figure 2 is a perspective view of an alternative hip spica cast undergarment according to another embodiment of the invention, provided with an perineal opening;
Figure 3 is an exploded view showing formation of the undergarment of Figure 1 or 2 by overlaying multiple sheets of padding material;
Figure 4 is an environmental view showing a padding undergarment in place on an infant; and
Figure 5 is a view showing the hip spica cast after cast tape has been applied over the undergarment padding material.

### Detailed Description of Preferred Embodiment of the Invention and Best Mode

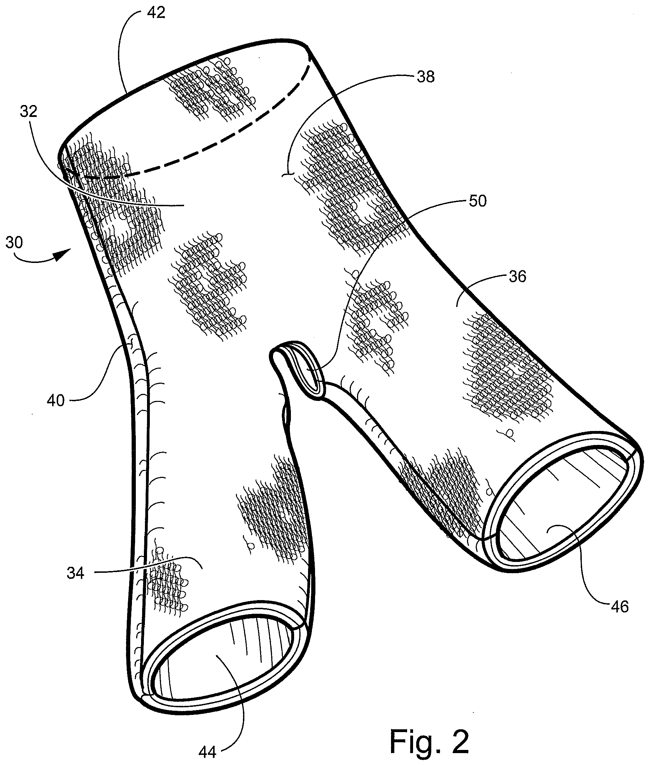

Referring now specifically to the drawings, a hip spica undergarment 10 is shown. The particular hip spica undergarment 10 is a pantaloon hip spica that extends from below the armpits of the patient to above the knees. The undergarment 10 includes a torso 12 and two integrally-formed legs 14 and 16 that are constructed of front and back blanks 18 and 20 of padding material. The patient is positioned in the undergarment 10 through a waist opening 22 with its legs extending through respective leg openings 24, 26. The undergarment 10 shown is seamed up the left and right sides and up the inside of each leg 14, 16 by ultrasonic welding of the synthetic material from which the blanks 18 and 20 are formed. One suitable material from which the undergarment 10 is constructed is a padding in sheet form that is presently sold in strips as Delta-Dry® brand synthetic padding manufactured and sold by BSN medical, Inc. and BSN medical, GmbH. Figure 2 illustrates a hip spica pantaloon undergarment 30, which includes a torso 32 and two integrally-formed legs 34 and 36 that are constructed of front and back sheets of Delta-Dry® brand synthetic padding material 38 and 40. The patient is positioned in the undergarment 30 through a top opening 42 with its legs extending through respective leg openings 44, 46. The undergarment 30 shown is seamed up the left and right sides and up the inside of each leg 34, 36 by ultrasonic welding of the synthetic material from which the blanks 38 and 40 are formed. The seam may also be formed by any suitable method, such as by butt or overedge seaming, or with an adhesive, either in tape or atomized glue form. Undergarment 30 includes a perineal opening 50, which may be preformed in the undergarment 30 when constructed, or may be cut out to a desired size, position and shape during application.

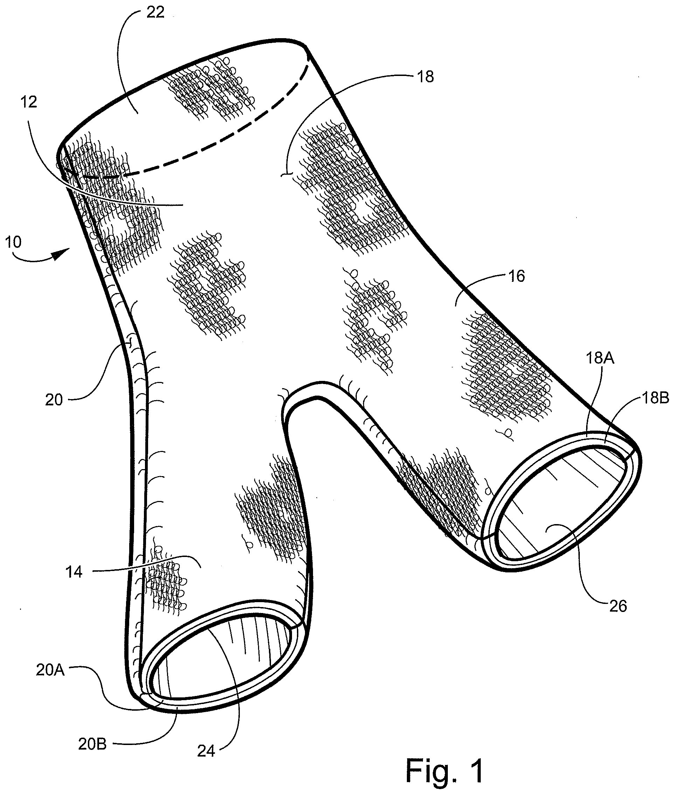

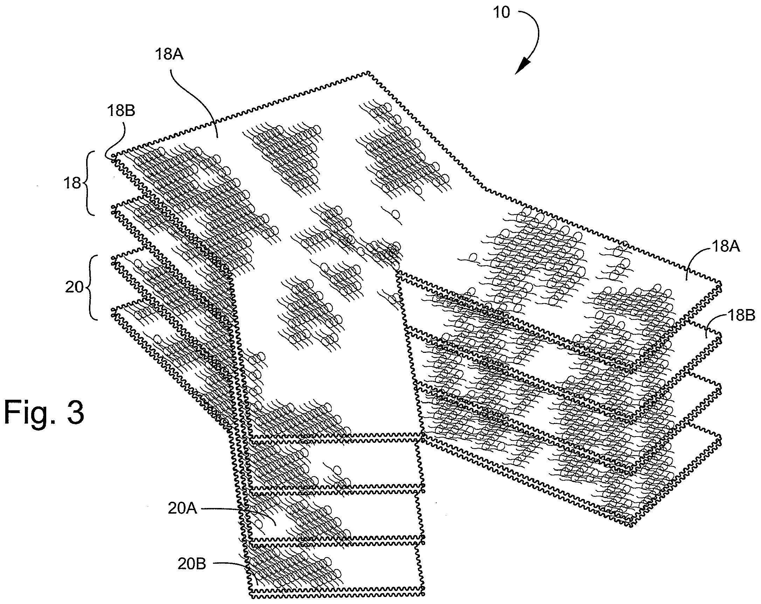

As shown in Figure 3 with reference to the undergarment 10 shown in Figure 1, each blank 18 and 20 are themselves preferably formed of two layers of padding material 18A, 18B, and 20A, 20B, such as Delta-Dry® brand synthetic padding. The double thickness provided by the layers 18A, 18B and 20A, 20B provide comfort and aids in protecting the skin from contact with the chemicals present on the cast tape applied over the undergarment 10. A double layer provides enhance comfort while still providing a moisture vapor transmission rate (MVTR) of over 1000 g/m²/24 hrs. Other embodiments may include single thickness or three or more thicknesses of the padding material.

The layers of padding shown in Figures 1, 2 and 3 can be constructed using any suitable hydrophobic/water resistant monofilament yarn such as polypropylene, polyester, polyethylene and nylon. The monofilament yarn used preferably has a diameter of at least 0.03 mm and is constructed with a 3D spacer fabric construction to provide sufficient cushioning and breathability, and it has been found that the use of a monofilament hydrophobic yarn on both faces and in the spacer area provides enhanced water resistance, light weight, breathability and resistance to collapse and degradation due to moisture and bacteria during extended use. Such a fabric is set out in US Published Application No. 2012/0220908, at paragraphs 0063-0068.

The padding material 18A, 18B, 20A, 20B can be constructed using any suitable organic or inorganic monofilament yarn, preferably a hydrophobic/water resistant monofilament yarn such as polypropylene, polyester, polyethylene and nylon. The monofilament yarn used for constructing the padding material preferably has a diameter of at least 0.03 mm. The padding material is constructed in a 3D spacer fabric construction to provide sufficient cushioning and breathability, and it has been found that the use of a monofilament hydrophobic yarn on both faces and in the spacer area provides enhanced water resistance, light weight, breathability and resistance to collapse and degradation due to moisture and bacteria during extended use.

The padding material is formed using any suitable fabric forming technology such as weaving, various knitting techniques such as, for example, weft knitting and warp knitting, non-woven, stitching, or a combination of these techniques. Preferably, the structure should provide some stretch in both the length-wise and width-wise directions, and facilitate conforming the undergarment 10 and 30 around an anatomical shape during application.

The padding material can be treated with one or more finishes to provide additional water resistance, anti-bacterial and/or anti-odor characteristics, or aromatherapy to improve the functionality or enhance the cast-wearing experience for the patient. Alternatively, the padding material can be fabricated from modified/treated monofilament yarns incorporating suitable fillers or finishes to improve the performance.

In one preferred embodiment, the undergarment 10 is constructed as a 3D spacer fabric using polypropylene monofilament and a low tack, pressure sensitive adhesive on one surface. The monofilament yarn has a diameter of at least 0.03 mm, and preferably between 0.05-0.25 mm. Preferably, the padding material requires no additional finish or water repellency treatment.

More specifically, one preferred embodiment of the padding material is constructed of a polypropylene monofilament yarn on a double needle bed knitting machine, and can be knitted on either a warp knitting Raschel machine or a Crochet knitting machine. The padding material is preferably constructed using a pillar and inlay stitch on the surfaces and a 3 or 5 needle V in the spacer area. The yarn has a diameter of 0.03-0.25 mm. The fabric for the padding material is formed with at least 50 courses per meter preferably 200-850 courses per meter. The padding material weighs between 50-400 g/m², and more preferably between 100-250 g/m². The padding material has a nominal thickness when not compressed or under tension of approximately 1.5-3.5 mm.

Alternatively, an undercast liner may be constructed as a spacer fabric with at least one of the yarns being a multifilament or spun yarn in order to provide even more patient comfort. The liner may be treated with suitable fluorochemical, silicone or other water repellant finish to improve drainage and provide faster drying.

The padding material may also be formed using any suitable fabric forming technology such as weaving, various knitting techniques such as, for example, weft knitting and warp knitting, non-woven, stitching, or a combination of these techniques. Preferably, the structure should provide some stretch in both the length-wise and width-wise directions, and facilitate conforming the undergarment 10 or 30 around the anatomical shape of the body torso and legs during application.

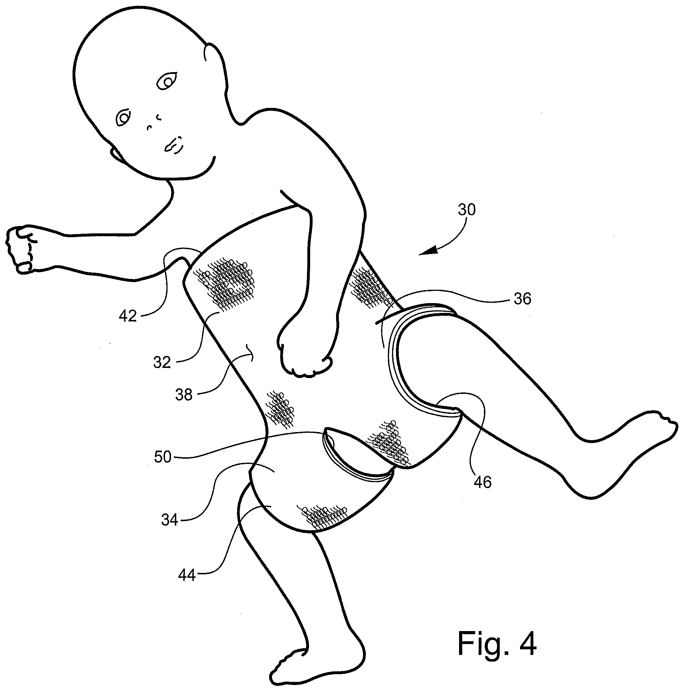

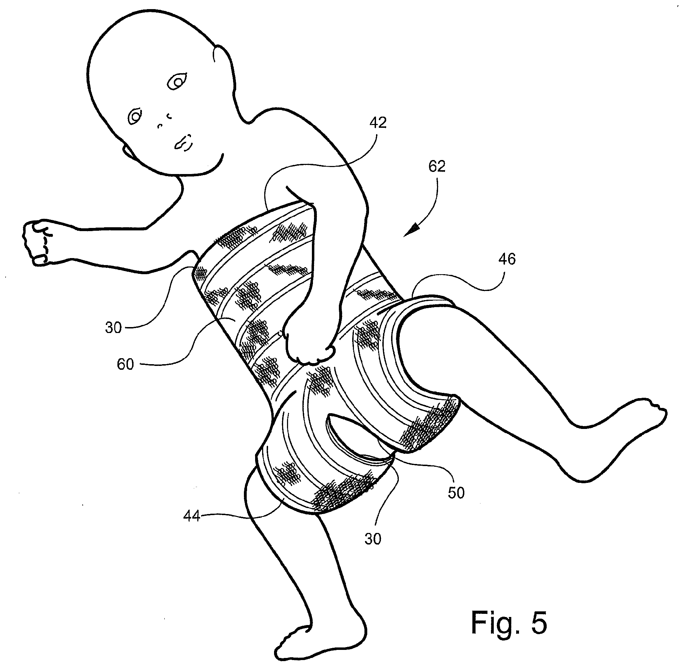

As noted above, Delta-Dry® brand synthetic padding is preferred. The fabric weight off of the machine and after a 60 minute relaxation period is 105 g/m². Referring now to Figures 4 and 5, and by way of example, the undergarment 30 of Figure 3 is applied to the infant by donning it over the legs and up across the torso to the medically-appropriate position-generally at or just below the armpits. As noted above, the stretch provided by the undergarment 30 permits a fast, accurate, closely-conforming application without wrinkles or creases. No stockinette or other material is used underneath the undergarment 30. It resides directly against the skin. The perineal opening 50 is either preformed in the undergarment 30 or formed during application.

As is shown in Figure 5, after application of the undergarment 30, a wrapping of synthetic cast tape 60 is applied. The preferred cast tape is Delta-Cast® brand synthetic fiber cast tape or Delta-Lite® brand fiberglass cast tape manufactured and sold by BSN medical, Inc. and BSN medical, GmbH. These cast tapes are supplied in various widths, the particular width being a choice left to the physician or cast technician. Two-inch or three-inch cast tape is the most often used. The cast tape 60 is removed from its moisture impervious pouch, wetted, and excess water removed by wringing the cast tape 60 while wrapped in an absorbent towel. The cast tape 60 is then applied over the undergarment 30 by wrapping the cast tape 60 circumferentially around the patient and applied to the injured limb, taking care in the usual manner to properly overlap adjacent wraps, eliminate wrinkles and puckers and leaving a short width of exposed undergarment at the top opening 42, leg openings 44 and 46, and perineal opening 50. The result is the cast 62 shown in Figure 5. The specific manner of application of the cast tape is a matter of medical procedure. One such procedure, illustrated with a prior art application of an underwrapping of strips of padding material, is contained in Application Manual-Hip Spica Cast, published by BSN medical, Inc., ©BSN medical, Inc., 2011, at pages 10-16.

The cast tape 60 hardens in several minutes to form a rigid cast 60 as shown in Figure 5 that is nevertheless very water and air permeable. Typically, the torso portion of the cast 60 can be covered in a soft cloth or garment, or the patient's arms covered in a soft garment, in order to prevent chafing of the patient's arms against the outer surface of the cast tape 60 on the torso portion of the cast 62.

For infant or small juvenile patients, the patient can be held in proximity to, for example, a handheld spray nozzle and the cast sprayed with a gentle spray of warm, cleansing water. Urine is readily washed away, and feces are dissolved, diluted and allowed to drain away. The patient is left clean and odor-free. Excess surface water is easily blotted away with an absorbent cloth or paper towel, and water on the interior evaporates within about one hour. This process can be repeated as necessary during the entire treatment period. Moreover, because of the ability to cleanse the patient as described above, it may not be necessary to provide the perineal opening in some instances.

An undergarment for use in forming a hip spica cast, and a hip spica cast according to the invention have been described with reference to specific embodiments and examples. Various details of the invention may be changed without departing from the scope of the invention. Furthermore, the foregoing description of the preferred embodiments of the invention and best mode for practicing the invention are provided for the purpose of illustration only and not for the purpose of limitation, the invention being defined by the claims.

## Claims

1. A water and air permeable undergarment (10, 30) for use with a cast tape (60) to form a hip spica cast (62), wherein the undergarment (10, 30) has a torso portion (12, 32) including a top opening (22, 42) for being positioned on a torso portion of the patient and at first and second leg portions (14, 16, 34, 36) terminating in respective leg openings (24, 26, 44, 46) adapted to reside between the hips and knees of the patient, **characterized in that** the undergarment (10, 30) is formed of first and second overlaid padding layers (18A, 18B) defining an anterior side of the undergarment (10, 30) and third and fourth overlaid padding layers (20A, 20B) defining a posterior side of the undergarment, wherein the first, second, third and fourth padding layers (18A, 18B, 20A, 20B) are layers of a water and air permeable 3D spacer synthetic padding material formed into a fabric of monofilament yarns and fabricated into an anatomically correct undergarment structure for being positioned around a torso and residing directly against the skin of a hip spica cast patient.

2. A water and air permeable undergarment (10, 30) according to claim 1, wherein the first and second padding layers (18A, 18B) and the third and fourth padding layers (20A, 20B) are joined along left and right seam lines extending from a top opening (22, 42) to first and second leg openings (24, 26, 44, 46) at a lowermost terminus of the undergarment.

3. A water and air permeable undergarment (10, 30) according to claim 2, wherein the monofilament yarns are thermoplastic and the seam lines are formed by a seaming process selected from the group consisting of ultrasonic energy, butt seaming, overedge seaming, joining with an adhesive tape and joining in with an atomized glue.

4. A water and air permeable undergarment (30) according to claim 1, wherein the undergarment (30) includes a perineal opening (50).

5. A water and air permeable undergarment (10, 30) according to claim 1, wherein the fabric for the padding material formed with at least 50 courses per meter preferably 200-850 courses per meter; and the padding material weighs between 50-400 g/m2 and has a nominal thickness when not compressed or under tension of approximately 1.5-3.5 mm.

6. A water and air permeable undergarment (10, 30) according to claim 5, wherein the padding material weighs between 100-250 g/m2.

7. A hip spica cast (60) for a patient, comprising:
(a) an undergarment (10, 30) according to any one of the claims 1 - 6;
(b) a water and air permeable synthetic cast tape (60) selected from the group consisting of a synthetic fiber or fiberglass/non fiberglass substrate coated or saturated with a moisture-curable resin and configured to be stored in a flexible condition in a moisture impervious package until use, such that removal of the cast tape (60) from the moisture impervious package and exposure to moisture causes the substrate to harden over a time period sufficiently long to allow application of the cast tape (60) while still flexible over the undergarment (10, 30); and
(c) the cast tape (60) positioned circumferentially over the undergarment (10, 30) and around the torso of the patient.

## Patentansprüche

1. Eine wasser- und luftdurchlässige Unterbekleidung (10, 30) zur Verwendung mit einem Gipsband (60) zur Bildung eines Becken-Beingipses (62), wobei die Unterbekleidung (10, 30) einen Rumpfabschnitt (12, 32) aufweist, mit einer oberen Öffnung (22, 42) zur Positionierung an einem Rumpfteil des Patienten und an ersten und zweiten Beinabschnitten (14, 16, 34, 36), die in entsprechenden Beinöffnungen (24, 26, 44, 46) enden, die eingerichtet sind, zwischen den Hüften und Knien des Patienten zu sitzen, **dadurch gekennzeichnet, dass** die Unterbekleidung (10, 30) aus ersten und zweiten übereinanderliegenden Polsterschichten (18A, 18B), die eine Vorderseite der Unterbekleidung (10, 30) definieren, und aus dritten und vierten übereinanderliegenden Polsterschichten (20A, 20B), die eine Rückseite der Unterbekleidung definieren, gebildet ist, wobei erste, zweite, dritte und vierte Polsterschichten (18A, 18B, 20A, 20B) Schichten aus einem wasser- und luftdurchlässigen 3D-Abstandshalter-Kunststoffpolstermaterial sind, das zu einem Gewebe aus Monofilamentgarnen geformt ist und in eine anatomisch korrekte Unterbekleidungsstruktur gefertigt ist, um um einen Torso herum positioniert zu werden und direkt an der Haut eines Becken-Beingips-Patienten zu liegen.

2. Wasser- und luftdurchlässige Unterbekleidung (10, 30) nach Anspruch 1, wobei die erste und zweite Polsterschicht (18A, 18B) und die dritte und vierte Polsterschicht (20A, 20B) entlang linker und rechter Nahtlinien verbunden sind, die sich von einer oberen Öffnung (22, 42) bis zu ersten und zweiten Beinöffnungen (24, 26, 44, 46) an einem unteren Ende der Unterbekleidung erstrecken.

3. Wasser- und luftdurchlässige Unterbekleidung (10, 30) nach Anspruch 2, wobei die Monofilamentgarne thermoplastisch sind und wobei die Nahtlinien durch ein Nahtverfahren gebildet sind, das aus der Gruppe ausgewählt ist, die aus Ultraschallenergie, Stumpfnähen, Überwendlichnähen, Verbindung mit einem Klebeband und Verbindung mit einem zerstäubten Klebstoff besteht.

4. Wasser- und luftdurchlässige Unterbekleidung (30) nach Anspruch 1, wobei die Unterbekleidung (30) eine Dammöffnung (50) enthält.

5. Wasser- und luftdurchlässige Unterbekleidung (10, 30) nach Anspruch 1, wobei das Gewebe für das Polstermaterial mit mindestens 50 Lagen pro Meter, vorzugsweise 200-850 Lagen pro Meter, gebildet ist; und wobei das Polstermaterial zwischen 50-400 g/m2 wiegt und eine Nominaldicke von etwa 1,5-3,5 mm hat, wenn nicht komprimiert oder unter Spannung.

6. Wasser- und luftdurchlässige Unterbekleidung (10, 30) nach Anspruch 5, wobei das Polstermaterial zwischen 100-250 g/m2 wiegt.

7. Ein Becken-Beingips (60) für einen Patienten, aufweisend:
(a) eine Unterbekleidung (10, 30) gemäß einem der Ansprüche 1 - 6;
(b) ein wasser- und luftdurchlässiges synthetisches Gipsband (60), ausgewählt aus der Gruppe bestehend aus einer synthetischen Faser oder einem Glasfaser-/nicht-Glasfasersubstrat, das mit einem feuchtigkeitshärtenden Harz beschichtet oder gesättigt ist, und eingerichtet ist, dass es bis zur Verwendung in einem flexiblen Zustand in einer feuchtigkeitsundurchlässigen Verpackung gelagert werden kann, so dass das Entfernen des gegossenen Bandes (60) aus der feuchtigkeitsundurchlässigen Verpackung und die Einwirkung von Feuchtigkeit bewirkt, dass das Substrat über einen ausreichend langen Zeitraum aushärtet, um das Anbringen des gegossenen Bandes (60) zu ermöglichen, während es über der Unterbekleidung (10, 30) noch flexibel ist; und
(c) das Gipsband (60), das in Umfangsrichtung über der Unterbekleidung (10, 30) und um den Rumpf des Patienten herum positioniert ist.

## Revendications

1. Sous-vêtement perméable à l'eau et à l'air (10, 30) pour une utilisation avec un bandage en plâtre (60) pour former un spica plâtré de hanches (62),
dans lequel le sous-vêtement (10, 30) a une partie de torse (12, 32) comportant une ouverture supérieure (22, 42) pour être positionnée sur une partie de torse du patient et au niveau des première et deuxième parties de jambes (14, 16, 34, 36) se terminant par des ouvertures de jambes respectives (24, 26, 44, 46) adaptées pour résider entre les hanches et les genoux du patient,
**caractérisé en ce que**
le sous-vêtement (10, 30) est formé de première et deuxième couches de rembourrage superposées (18A, 18B) définissant un côté antérieur du sous-vêtement (10, 30) et des troisième et quatrième couches de rembourrage superposées (20A, 20B) définissant un côté postérieur du sous-vêtement, où les première, deuxième, troisième et quatrième couches de rembourrage (18A, 18B, 20A, 20B) sont des couches d'un matériau de rembourrage synthétique d'espacement 3D perméable à l'eau et à l'air formées en un tissu de fils monofilaments et fabriquées en une structure de sous-vêtement anatomiquement correcte pour être positionnée autour d'un torse et résidant directement contre la peau d'un patient portant un spica plâtré de hanches.

2. Sous-vêtement perméable à l'eau et à l'air (10, 30) selon la revendication 1, dans lequel les première et deuxième couches de rembourrage (18A, 18B) et les troisième et quatrième couches de rembourrage (20A, 20B) sont jointes le long des lignes de couture gauche et droite s'étendant d'une ouverture supérieure (22, 42) aux première et deuxième ouvertures de jambes (24, 26, 44, 46) au niveau de l'extrémité la plus basse du sous-vêtement.

3. Sous-vêtement perméable à l'eau et à l'air (10, 30) selon la revendication 2, dans lequel les fils monofilaments sont thermoplastiques et les lignes de couture sont formées par un procédé de couture choisi dans le groupe constitué de l'énergie ultrasonique, de la couture bout à bout, de la couture à surjet, de l'assemblage par un ruban adhésif et de l'assemblage par une colle atomisée.

4. Sous-vêtement perméable à l'eau et à l'air (30) selon la revendication 1, dans lequel le sous-vêtement (30) comporte une ouverture périnéale (50).

5. Sous-vêtement perméable à l'eau et à l'air (10, 30) selon la revendication 1, dans lequel le tissu pour le matériau de rembourrage étant formé d'au moins 50 rangées de mailles par mètre, de préférence entre 200 et 850 rangées de mailles par mètre ; et
le matériau de rembourrage pèse entre 50 et 400 g/m² et a une épaisseur nominale allant d'environ 1,5 à 3,5 mm lorsqu'il n'est pas comprimé ou soumis à une tension.

6. Sous-vêtement perméable à l'eau et à l'air (10, 30) selon la revendication 5, dans lequel le matériau de rembourrage pèse entre 100 et 250 g/m².

7. Spica plâtré de hanches (60) pour un patient, comprenant :
(a) un sous-vêtement (10, 30) selon l'une quelconque des revendications 1 à 6 ;
(b) un bandage en plâtre synthétique perméable à l'eau et à l'air (60) choisi dans le groupe constitué d'un substrat en fibres de verre/sans fibres de verre ou en fibres synthétiques revêtu ou saturé d'une résine durcissable à l'humidité et configuré pour être stocké dans un état flexible dans un emballage étanche à l'humidité jusqu'à son utilisation, de sorte que le retrait du bandage en plâtre (60) de l'emballage étanche à l'humidité et son exposition à l'humidité amènent le substrat à durcir pendant une période suffisamment longue pour permettre l'application du bandage en plâtre (60) tout en restant flexible sur le sous-vêtement (10, 30) ; et
(c) le bandage en plâtre (60) positionné de manière circonférentielle sur le sous-vêtement (10, 30) et autour du torse du patient.
